# EUROPEAN PATENT SPECIFICATION

(11) **EP 3 403 493 B1**
(45) Date of publication and mention of the grant of the patent: **12.05.2021**
(21) Application number: 18182593.6
(22) Date of filing: 19.10.2011
(51) Int. Cl.: A01K 29/00, A61D 1/16, A61B 5/00, G01S 5/02, A61B 17/52, A61D 7/00, A61B 5/07, A01K 11/00

(54) **METHOD OF MONITORING AN ANIMAL**
TIERÜBERWACHUNGSVERFAHREN
PROCÉDÉ DE SURVEILLANCE D'UN ANIMAL

(30) Priority: 19.10.2010 US 455419 P
(43) Date of publication of application: 21.11.2018
(62) Divisional of application: 11834759.0
(73) Proprietor: ST Reproductive Technologies, LLC, Navasota, TX 77868 (US)
(72) Inventor: RETTEDAL, Nicholas P., Berthoud, CO 80513 (US); WEILNAU, Stephen M., Greeley, CO 80634 (US); COCKROFT, Scott R., Greeley, CO 80631 (US); YEAGER, Billy J., Gilbert, AZ 85297 (US); HORNICK, Jerry A., Gold Canyon, AZ 85118 (US)
(74) Representative: Kador & Partner PartG mbB

(56) References cited:
- US-A- 5 984 875
- US-A- 6 085 751
- US-A1- 2004 155 782

## Description

### I. TECHNICAL FIELD

Generally, a method of monitoring an animal by use of a monitoring device configured as a bolus for oral administration to reside in an animal's stomach. The bolus has a substantially inert solid body which contains within an animal monitoring device. The animal monitoring device includes a radio frequency generator, an animal identification information encoder for outputting animal identification information of the particular animal. The animal monitoring device can further include sensors to detect one or more physiological and non-physiological sensed animal characteristics and a sensed animal characteristic encoder for outputting sensed animal characteristic information. The animal monitoring device intermittently transmits encoded animal identification information and encoded sensed animal characteristic information to a radio frequency reader which assembles and transmits encoded information as data packets to a reception device which allows a specialized computer to display decoded animal identification information and decoded sensed animal characteristic information as numeric values which can be accessed by a user.

### II. BACKGROUND

A variety of animal monitoring devices are in use to remotely track animal location and remotely sense the temperature of animals. US5,984,875 A and US2004/155782 A1 disclose such exemplary monitoring devices of the prior art. Certain of these devices include an orally administered, inserted, or ingested bolus containing microprocessors for processing animal identification information and signals from sensors to provide encoded data representations which can be transmitted by radio-frequency to a radio-frequency receiver. However, certain problems remain unresolved which relate to the structure and function of the bolus electrical circuitry and the transmission of encoded data representations by these conventional animal monitoring devices.

One problem related to conventional bolus may be that there is no magnet located within the bolus which generates a magnetic field to collect metal materials ingested by the animal such as wire, nails, screws, tacks, barbed wire, or the like. Alternately, conventional bolus may contain within one or more magnets, but the magnetic field generated may dispose attracted metal elements in an orientation which projects outwardly from the bolus. These projecting metal elements can cause injury to the animal.

Another problem related to conventional bolus can be that the magnet has a location sufficiently close to or as a part of the components generating the radio-frequency which carries encoded data representations generated by the microcontroller or processor elements resulting in loss of encoded data representations during transmission to the radio frequency receiver.

Another problem related to conventional bolus may be that the mass of the animal in which the bolus has a location can demodulate the frequency of the radio signal such that the radio signal has a different frequency at the point of transmission than the frequency of the radio signal after passing through the mass of the animal. Accordingly, encoded data representations can be intermittently interrupted or portions or all of the transmitted encoded data representations can be lost.

As to each of these substantial problems, the animal monitoring system described herein provides a solution.

### III. DISCLOSURE OF INVENTION

Accordingly, a broad object of the invention can be to provide a method of monitoring an animal by use of a bolus, which is orally administratable for retention in the digestive tract of an animal which contains an animal monitoring device having a structure and a function which improves transmission of encoded animal identification information and encoded sensed animal characteristic information from within an animal to a radiofrequency reader.

Another broad object of the invention can be to provide a method of monitoring an animal by use of a bolus which includes one or more magnets disposed to generate one or more magnetic fields having a configuration which attracts metal objects to the external surface of the body of the bolus but avoids disposing such metal objects in outwardly projecting relation the external surface of the body of the bolus.

Another broad object of the invention can be to provide a method of monitoring an animal by use of an animal monitoring device on a printed circuit board which can be sufficiently isolated from the one or more magnets to allow transmission of encoded animal identification information and sensed animal characteristic information without interruption or loss of encoded information. Another broad object of the invention can be to provide a network frequency match element which functions as part of the animal monitoring device to compensate for the mass of the animal such that the radiofrequency signal generated by the animal monitoring device antenna located inside the animal can be received by the radio frequency reader antenna located outside of the animal.

The invention provides a method of monitoring an animal as claimed in independent claim 1. Preferred embodiments of the invention are set out in the dependent claims.

Naturally further objects of the invention are disclosed throughout the detailed description of the preferred embodiments of the invention and the figures.

### IV. BRIEF DESCRIPTION OF THE DRAWINGS

Figure 1 is diagram which shows a particular method of using an embodiment of the animal monitoring system.
Figure 2 is a block diagram which shows a particular embodiment of a specialized computer in relation to a particular embodiment of a radio frequency reader and bolus.
Figure 3 is a block diagram which shows a particular embodiment of a radio frequency reader.
Figure 4 is an exploded view of a particular embodiment of the bolus.
Figure 5 is an exploded view of another particular embodiment of the bolus.
Figure 6 is block diagram of a particular embodiment of the animal monitoring device which can be contained in a various embodiments of the bolus.
Figure 7 is a bar graph which compares strength of radio frequency transmission against orientation of magnetic field of a first magnet contained in bolus.
Figure 8 is a bar graph which compares strength of radio frequency transmission against orientation of magnetic field of a first magnet contained in the bolus when magnetically coupled to a second magnet outside of the bolus.
Figure 9 is a bar graph which compares strength of radio frequency transmission with the first magnet contained in the bolus oriented to provide greatest strength of radio frequency transmission as compared to strength of radio frequency transmission with the first magnet contained in the bolus oriented to provide greatest strength of radio frequency transmission with a second magnet outside of the bolus magnetically coupled to the first magnet.

### V. MODE(S) FOR CARRYING OUT THE INVENTION

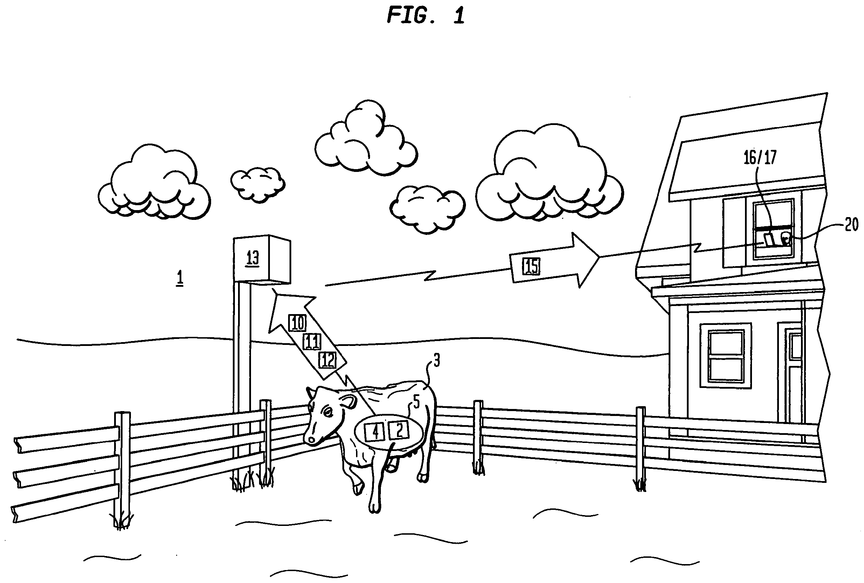

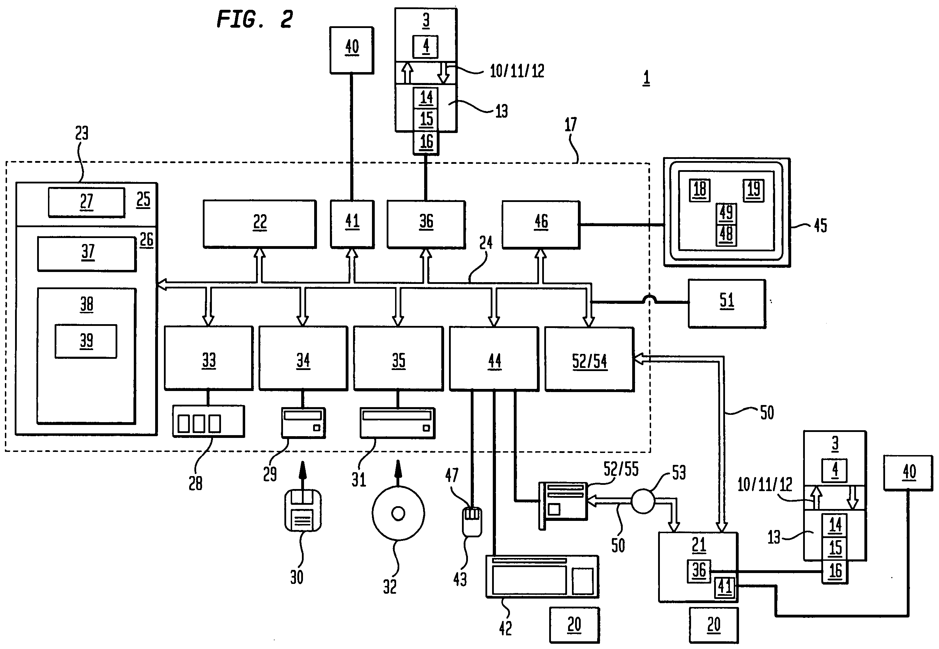

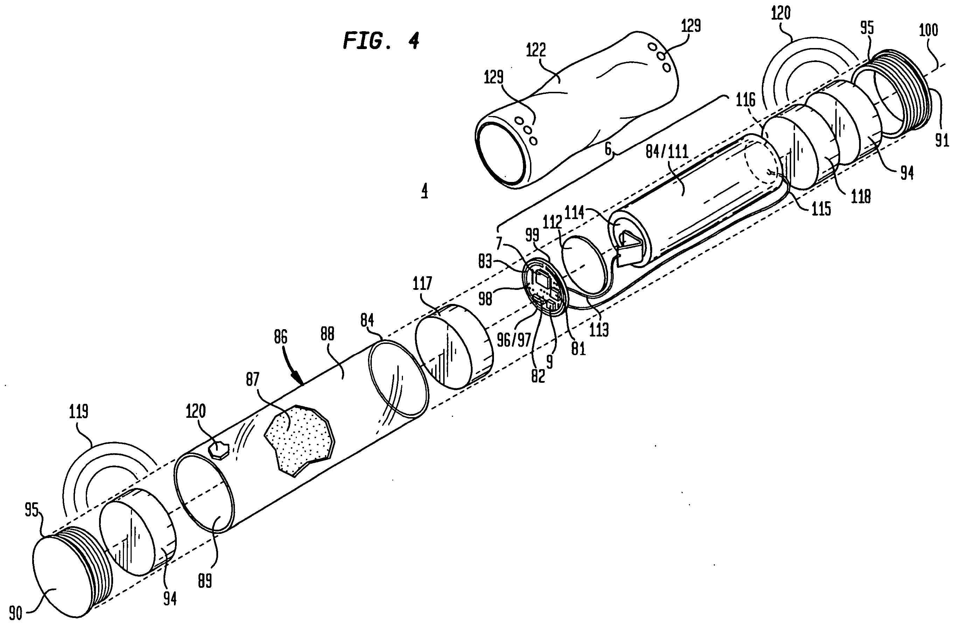

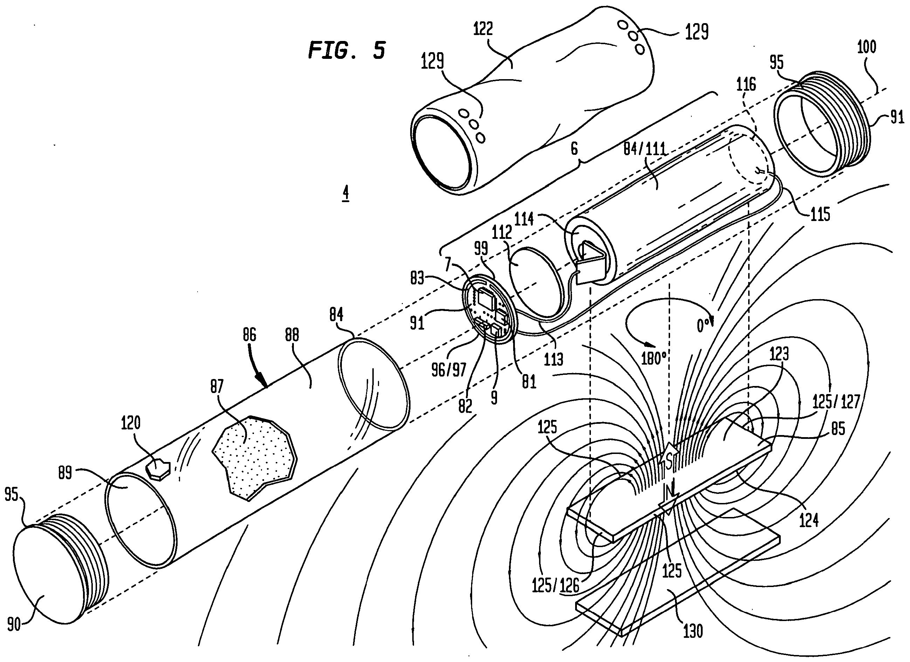

Now referring primarily to Figures 1 and 2, which illustrates a general computer implemented method of using an animal monitoring system (1) to monitor one or more sensed physiological and non-physiological parameters ("animal characteristics (2)") of an animal (3). A bolus (4) can be orally administered to reside in a reticulum (5) of the animal (3) (although the bolus (4) can be implanted in the animal (3) to reside at other locations). The bolus (4) can include an animal monitoring device (6) (see for example Figures 4 and 5) including one more sensors (9) which can sense animal characteristics (2). A microcontroller (7) having one or more processors (8) continually or intermittently transform analog or digital signals from the one or more sensors (9) to generate encoded sensed animal characteristic information (10). The encoded sensed characteristic information (10) varies in relation to monitored change in the sensed animal characteristics (2). The animal monitoring device (6) can further generate encoded animal identification information (11) associated the individual monitored animal (3). The animal monitoring device (6) can further operate to generate and transmit a radio frequency signal (12) (also referred to as an "RF signal") which can carry encoded animal identification information (11) and encoded sensed animal characteristic information (10).

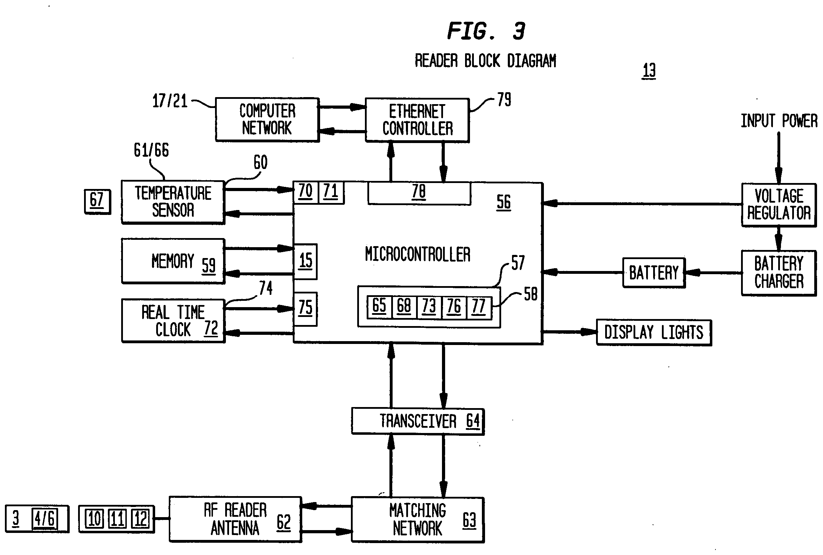

Again referring primarily to Figures 1 and 2, one or more radio frequency reader(s) (13) can be located to receive the radiofrequency signal (12) carrying the encoded animal identification information (1 I) and the encoded sensed animal characteristic information (10). As to particular embodiments, the one or more radiofrequency readers (13) can further operate to decode the received radiofrequency signal (12) and generate one or more bit segments (14) representing the encoded animal identification information (1 I) and representing the encoded sensed animal characteristic information (10) (see for example Figure 3). As to particular embodiments, the one or more radio frequency readers (13) can further operate to assemble the bit segments (14) into a data packet (15) which can be transmitted and received by a wired or wireless reception device (16). The reception device (16) can transfer the data packet (15) to a specialized computer (17) for transforming the bit segments (14) to output an animal identification value (18) and to output a sensed animal characteristic value (19). A computer user (20) can access the sensed animal characteristic value (19) associated with the animal identification value (18) (along with other information encoded by the animal monitoring device (6) or the radio frequency reader (13) or a remote second computer (21)) by use of a specialized computer (17).

Now referring primarily to Figure 2, the specialized computer (17) configured to allow access by the computer user (20) of the sensed animal characteristic values (19) associated with the animal identification value (18) is described herein in terms of functional block components, screen shots, and various process steps. It should be appreciated that such functional blocks may be realized by any number of hardware or software components configured to perform the specified functions. For example, the computer implemented animal management system (I) may employ various integrated circuit components which function without limitation as: memory elements, radio frequency signal modulators, processing elements, logic elements, look-up tables, or the like, which may carry out a variety of functions under the control of one or more microprocessors or other control devices.

Similarly, the software elements of the present invention may be implemented with any programming or scripting language such as C, C++, Java, COBOL, assembler, PERL, Labview or any graphical user interface programming language, extensible markup language (XML), Microsoft's Visual Studio .NET, Visual Basic, or the like, with the various algorithms or Boolean Logic being implemented with any combination of data structures, objects, processes, routines or other programming elements. Further, it should be noted that the present invention might employ any number of conventional wired or wireless techniques for data transmission, signaling, data processing, network control, and the like.

It should be appreciated that the particular computer implementations shown and described herein are illustrative of the invention and its best mode and are not intended to otherwise limit the scope of the present invention in any way. Indeed, for the sake of brevity, conventional data networking, application development and other functional aspects of the systems (and components of the individual operating components of the systems) may not be described in detail herein. Furthermore, the connecting lines shown in the various figures contained herein are intended to represent exemplary functional relationships and/or physical couplings between the various elements. It should be noted that many alternative or additional functional relationships or physical connections may be present in a practical animal monitoring system (1).

As will be appreciated by one of ordinary skill in the art, the present invention may be embodied in the alternative as a method, a data processing system, a device for data processing, a computer program product, or the like. Accordingly, the present invention may take the form of an entirely software embodiment, an entirely hardware embodiment, or an embodiment combining aspects of both software and hardware. Furthermore, the present invention may take the form of a computer program product on a computer-readable storage medium having computer-readable program code means embodied in the storage medium. Any suitable computer-readable storage medium may be utilized, including hard disks, CD-ROM, optical storage devices, magnetic storage devices, ROM, flash RAM, or the like.

The present invention may be described herein with reference to screen shots, block diagrams and flowchart illustrations of the data encoder-decoder system to describe computer programs, applications, or modules which can be utilized separately or in combination in accordance with various aspects or embodiments of the invention. It will be understood that each functional block of the block diagrams and the flowchart illustrations, and combinations of functional blocks in the block diagrams and flowchart illustrations, respectively, can be implemented by computer program instructions. These computer program instructions may be loaded onto a general purpose computer, special purpose computer or other programmable data processing apparatus to produce a machine, such that the instructions which execute on the computer or other programmable data processing apparatus for implementing the functions specified in the flowchart block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions which execute on the computer or other programmable apparatus provide steps for implementing the functions specified in the flowchart block or blocks.

Accordingly, functional blocks of the block diagrams and flowchart illustrations support combinations of means for performing the specified functions, combinations of steps for performing the specified functions, and program instruction means for performing the specified functions. It will also be understood that each functional block of the block diagrams and flowchart illustrations, and combinations of functional blocks in the block diagrams and flowchart illustrations, can be implemented by either special purpose hardware based computer systems which perform the specified functions or steps, or suitable combinations of special purpose hardware and computer instructions.

Again referring to Figure 2, the computer implemented animal monitoring system (1) can include a specialized computer (17) for receiving, processing and transforming signals from a reception device (16) to generate animal identification values (18) and sensed animal characteristic values (17) accessible by the computer user (20)). The specialized computer (17) can include at least one processing unit (22), a memory element (23), and a bus (24) which operably couples components of the computer (17), including, without limitation the memory element (23) to the processing unit (22). The computer (17) may be a conventional computer, a distributed computer, or any other type of computer which may contain all or a part of the elements described or shown to accomplish the functions described herein; the invention is not so limited. The processing unit (22) can comprise without limitation one central-processing unit (CPU), or a plurality of processing units which operate in parallel to process digital information, or a digital signal processor (DSP) plus a host processor, or the like. The bus (24) can be without limitation any of several types of bus configurations such as a memory bus or memory controller, a peripheral bus, and a local bus using any of a variety of bus architectures. The memory element (23) can without limitation be a read only memory (ROM) (25) or a random access memory (RAM) (26), or both. A basic input/output system (BIOS) (27) containing routines that assist transfer of data between the components of the specialized computer (17), for example during start-up, can be stored in ROM (25). The computer (17) can further include a hard disk drive (28) for reading from and writing to a hard disk (not shown), a magnetic disk drive (29) for reading from or writing to a removable magnetic disk (30), and an optical disk drive (31) for reading from or writing to a removable optical disk (32) such as a CD ROM or other optical media.

The hard disk drive (28), magnetic disk drive (29), and optical disk drive (31) and the reception device (16) can be connected to the bus (24) by a hard disk drive interface (33), a magnetic disk drive interface (34), and an optical disk drive interface (35), and a reception device interface (36), respectively. The drives and their associated computer-readable media provide nonvolatile storage of computer-readable instructions, data structures, program modules and other data for the computer (17). It can be appreciated by those skilled in the art that any type of computer-readable media that can store data that is accessible by a computer, such as magnetic cassettes, flash memory cards, digital video disks, Bernoulli cartridges, random access memories (RAMs), read only memories (ROMS), RFID devices or the like, may be used in the exemplary operating environment.

The computer (17) can further include an operating system (37) and an animal monitoring program (38) (AMP) which as to particular embodiments of the invention can include an animal monitoring device encoder-decoder module (39) (AMD encoder-decoder module) for programming animal identification values (18) to the animal monitoring device (AMD) (6) using an animal monitoring device programmer (40) connected to the bus (24) by an AMD interface (41). The AMD encoder-decoder module can be stored on or in the hard disk, magnetic disk (30), optical disk (32), ROM (25), in RAM (26) the specialized computer (17) or alternately the functionalities of the AMD encoder-decoder module (39) may be implemented as an application specific integrated chip (ASIC) or file programmable gate array (FPGA), or the like.

The computer user (20) can enter commands and information into the computer (17) through input devices such as a keyboard (42) and a pointing device (43) such as a mouse. Other input devices (not shown) may include a microphone, joystick, game pad, satellite dish, scanner, magnetic strip of a card, or the like. These and other input devices are often connected to the processing unit (22) through a serial port interface (44) that can be coupled to the bus (24), but may be connected by other interfaces, such as a parallel port, game port, or a universal serial bus (USB). A monitor (45) or other type of display device can also be connected to the bus (24) via interfaces such as a video adapter (46), or the like. In addition to the monitor (45), the computer (17) can further include a peripheral output device (51), such as speakers and printers.

A "click event" occurs when the computer user (20) operates at least one function of the AMP (38) or the animal monitoring device encoder-decoder module (39), or other program or other application function, through an action or the use of a command which for example can include pressing or releasing a left mouse button (47) while a pointer element (48) is located over a control icon (49) displayed on the monitor (45). However, it is not intended that a "click event" be limited to the press and release of the left mouse button (46) while a pointer element (45) is located over a control icon (49). Rather, the term "click event" is intend to broadly encompass any action or command by the computer user (20) through which a function of the operating system (37) or animal monitoring program (38), animal monitoring device encoder-decoder module (39), or other program or application is activated or performed, whether through clickable selection of one or a plurality of control icon(s) (49) or by computer user (20) voice command, keyboard stroke(s), mouse button, touch screen, touch pad, or otherwise. It is further intended that control icons (49) can be configured without limitation as a point, a circle, a triangle, a square (or other geometric configurations or combinations or permutations thereof), or as a check box, a drop down list, a menu, or other index containing a plurality of selectable options, an information field which can contain or which allows input of a string of alphanumeric characters such as a street address, zip code, county code, or natural area code, animal identification number or by inputting a latitude/longitude or projected coordinate X and Y, animal pen number, or other notation, script, character, or the like.

The computer (17) may operate in a networked environment using logical connections (50) to one or a plurality of remote second computers (21). These logical connections (50) can be achieved by a communication device (52) coupled to or a part of the computer (17). Each of the plurality of remote computers (51) can include a part or all of the elements as included in the specialized computer (17) although only a single box has been illustrated in Figure 2 for the remote second computer (51). The logical connections (50) depicted in Figure 2 can establish a local-area network (LAN) or a wide-area network (WAN). Such networking environments are commonplace in offices, enterprise-wide computer networks, intranets and the Internet (53).

When used in a LAN-networking environment, the computer (17) can be connected to the local network through a network interface (54). When used in a WAN-networking environment, the computer (17) typically includes a modem (55), or other type of communications device, for establishing communications over the wide area network, such as the Internet (53). The modem (55), which may be internal or external to the specialized computer (17), can be connected to the bus (24) via the serial port interface (44). In a networked environment, the animal monitoring program (38), or portions thereof, may be stored in any one or more of the plurality of remote second computers (51). It is appreciated that the logical connections (50) shown are exemplary and other hardware means and communications means can be utilized for establishing a communications link between the specialized computer (17) and one or more of the a plurality of remote second computers (21).

While the computer means and the network means shown in Figure 2 can be utilized to practice the invention including the best mode, it is not intended that the description of the best mode of the invention or any preferred embodiment of the invention be limiting with respect to the utilization of a wide variety of similar, different, or equivalent computer means or network means to practice embodiments of the invention which include without limitation hand-held devices, such as personal digital assistants or camera/cell phone, multiprocessor systems, microprocessor-based or programmable consumer electronics, network PCs, minicomputers, mainframe computers, PLCs, or the like.

Now referring primarily to Figures 1 and 3, the animal monitoring system (1) can further include one or more radio frequency readers (13)(RF readers). The RF reader (13) can receive a radio-frequency signal (12) from an AMD (6) within a bolus (4) implanted in, retained by, or held in the reticulum (5) of an animal (3). The AMD (6) within the bolus (4) can send encoded animal identification information (10) and the encoded sensed animal characteristic information (2) using the radio-frequency signal (12), as above described.

One non-limiting embodiment of the RF reader (13) as shown in Figures 1 and 3, provides a reader microcontroller (56) which includes a reader processor (57) which controls the functions of a variety of reader processor elements (58) stored in a reader memory element (59) each of which provides a response to events related to receiving the radio-frequency signal (12) from the AMD (6) within the bolus (4) carrying encoded animal identification information (1 1) and sensed animal characteristic information (10), or receiving reader sensor signals (60) from reader sensors (61) which monitor environmental parameters proximate the RF reader (13) such as ambient temperature; or generating data packets (15) which include all or parts of such information, or sending data packets (15) to the computer (17) or a remote second computer (21) for access by a computer user (20). A reader microcontroller (56) suitable for use with embodiments of the RF reader (13) can be obtained from Microchip Technology. Inc., 2355 West Chandler Blvd., Chandler, Arizona, Part No. P1C18F4620-1/PT, or similar or equivalent components can be suitable as a reader microcontroller (56) programmable to perform the above-described functions of the RF reader (13).

Again referring primarily to Figure 3, a reader antenna (62) can receive encoded animal identification information (10) and encoded sensed animal characteristic information (11) and other information generated by operation of the AMD (6) within the bolus (4) within an animal (3). The reader antenna (62) can be tuned to the radio-frequency signal (12) generated by the AMD (6) by a reader matching network element (63). A reader receiver (64) (or transceiver) can be controlled by a first reader processor element (65) to convert the radio-frequency signal (12) received by the reader antenna (62) from analog to digital baseband signals.

Again referring primarily to Figure 3, the reader sensor (61) can take the form of an ambient temperature sensor (66) which can be located to sense the ambient temperature (67) surrounding the RF reader (13). The ambient temperature sensor (66) can take the form of a thermistor. A suitable thermistor for use in embodiments of the RF reader (13) is available from Microchip Technology, Inc., 2355 West Chandler Blvd., Chandler, Arizona, Part No.MCP98242, and similar and equivalent parts. The ambient temperature sensor (66) can be operated under the control a second reader processor (68) which functions to regulate power to the ambient temperature sensor (66) and converts the reader sensor signal (60) from the ambient temperature sensor (66) into a digital representation of the ambient temperature (67). The second reader processor (68) can further function to encode or re-encode from time to time an amount of reader temperature calibration data (70) which allows calculation and output of an ambient temperature value (71).

Again referring primarily to Figure 3, a clock element (72) can operate under the control of a third reader processor element (73) which functions to generate a date and time signal (74) that represents a date and time value (75).

Again referring primarily to Figure 3, a fourth reader processor element (76) can function to assemble data packets (15) which include a representation of, the ambient temperature value (71) and the date and time value (75) at which the information from the AMD (6) was received by the RF reader (13). The assembled data packet (15) can be stored and retrieved from the reader memory element (59) under the control of the fourth reader processor element (76).

Again referring primarily to Figure 3, a fifth reader processor element (77) can function to provide an ether net interface (78) for an ether net controller (79) to receive requests from the computer (17) (or remote computer (21) and retrieve from the reader memory element (59) one or more data packets (15) containing information relating to one or a plurality of animals (3) which entrain a bolus (4) with an AMD (6). The fifth reader processor element (77) can further function to send the retrieved data packets (15) to the ether net controller (79) for transmission to the computer (17).

Now referring primarily to Figures 4 through 6, the animal monitoring system (1) includes an inert bolus (4) orally administrable to an animal (or implantable in an animal) (3) containing the AMD (6) which includes one or more of a microcontroller (7), one or more processors (8), at least one sensor (9), and a radio frequency generator (81) including one or more of an oscillator (80), a radio frequency stabilizer (82), an antenna (83), and a power source (84) which operate to generate the radio frequency signal (12).

A first magnet (85) (see example shown in Figure 5) or optionally a pair of magnets (94) (see example shown in Figure 4) are further included in the inert bolus (4). The bolus (4) is orally administered to ruminate animals (3), such as cows, deer, and sheep, and can be retained in a part of the stomach, such as the reticulum (5), as shown in Figure 1; and in non-claimed embodiments of the bolus (4) can be implanted or be otherwise affixed to an animal (3).

Embodiments of the bolus (4) which are orally administered to an animal (3) can provide an inert bolus body (86) having external dimensional relations adapted to allow oral administration and retention of the bolus (4) in a part of the stomach, such as the reticulum (5) of a particular species of animal (3). As one non-limiting example, the inert bolus body (86) can include an amount of cured plastic resin (87) cast about the animal monitoring device (6) and as to particular embodiments about the pair of magnets (94) or the first magnet (85) along with any spacers. The amount of cured plastic resin (87) can for example comprise a plastic resin such as urethane resin, epoxy resin, polyester resin, or the like used in accordance with the manufacturer's instructions. As to other embodiments, the inert bolus body (86) can comprise a scalable container (88) which defines a hollow inside space (89) which receives said animal monitoring device (6) and said first magnet (85). As to other embodiments, the sealable container having the animal monitoring device (6) received in the hollow space (89) (and as to particular embodiments further including the first magnet (85) received in the hollow space) can have the amount of plastic resin (87) cast about the animal monitoring device (6) and the first magnet (85) located within said sealable container (88).

As one illustrative example, a bolus (4) suitable for oral administration to an animal (3) can be generally cylindrical with a diameter in perpendicular cross section in the range of about one-half inch to about one inch and having a length disposed between a first bolus end (90) and a second bolus end (91) in the range of about two inches and about five inches. Particular embodiments of the bolus (4) can have a length of about three and one-half inches and a diameter in perpendicular cross section of about three-quarters of an inch. While the Figures show the bolus (4) in the constructional form of a cylinder with end caps; the invention is not so limited, and the bolus (4) can have numerous and varied external surface configurations which allow oral administration and retention within the reticulum (5) (or other part of the digestive tract) of an animal (3). Typically, retention of the bolus (4) in a part of a stomach or retention by way of implant will be for all or a substantial portion of the life of the animal (3). The inert bolus body (86) can be molded, cast, or machined from biocompatible (or biologically inert) non-magnetic materials which allow transmission of the radio frequency signal (12) from within the bolus (4) to outside of the animal (3). As examples, the inert bolus body can be made from plastics such as nylon, fluorocarbon, polypropylene, polycarbonate, urethane, epoxy, polyethylene, or the like; or metals such as stainless steel; or other materials such as glass can be utilized.

The hollow inside space (89) inside of the inert bolus body (86) can be of sufficient volume to house one or more of the microcontroller (7), the sensor (9), the oscillator (80) the radio frequency stabilizer (82), the antenna (83) and the power source (84) along with the associated circuitry. Now referring primarily to Figure 4, as to certain embodiments of the bolus body (86), the hollow inside space (89) can have sufficient volume to further house nonconductive insulators (92), and non-conductive spacers (93) to establish a particular distance between a pair of magnets (94), while as to embodiments of the invention similar to that shown in Figure 5, the hollow inside space (89) can have sufficient volume to further house a first magnet (85). As to embodiments of the bolus (4) as shown in Figures 4 and 5 or similar embodiments, the hollow inside space (89) can be configured as a cylindrical volume having a diameter of about three-eighths of an inch and about five-eighths inch and a length disposed between the first bolus end (90) and the second bolus end (91) of between about two inches and about four inches. A particular non-limiting embodiment of the hollow inside space (89) can be about one-half inch in diameter and having a length of about three inches.

As to those embodiments of the bolus (4) including a sealable container (88), as above described, the sealable container (88) can further provide at least one end cap (95) removably sealable with a first bolus end (90) or a second bolus end (91) or both ends (90)(91) of the bolus (4) to allow access to the hollow inside space (89) for location of the various components of the animal monitoring device (6). As to certain embodiments of the invention, the bolus (4) can take the form of a closed end tube having one end cap (95) or a cylindrical tube having an end cap (95) fitted to each of the first bolus end (90) and the second bolus end (92). The end cap(s) (95) can also take the form of a plug sealably inserted into one or both ends of the sealable container (88), as shown in Figures 4 and 5. Alternately, the end cap (95) and the bolus (4) can provide rotatably matable spiral threads. Additionally, the end cap (95) can take the form of a permanent seal to one or both ends of the sealable container (88) of the bolus (4) such as a castable polymer which cures to seal one or both ends of the bolus (4). The bolus (4) can also take the form of matable halves (whether longitudinal or latitudinal) which can avoid the use of end caps (95).

The bolus (4) having a hollow inside space (89) can be generated by a wide variety of procedures such as molding, casting, fabrication or the like. As one non-limiting example, a cylindrical tube having an external diameter and an internal diameter, as above described, can be divided into sections of suitable length to which the end caps can be fitted. Alternately, a bore can be made in a cylindrical solid rod having an external diameter, as above described, to provide a closed end tube with the bore having sufficient dimension to provide the hollow inside space (89). An end cap (95) or seal can be fitted to the open end of the closed end tube.

Now referring primarily to Figures 4 through 6, a printed circuit board (96) can be utilized to mechanically support and electrically connect the microcontroller (7), the sensor (9), the oscillator (80), the radio frequency stabilizer (82), and the antenna (83). The printed circuit board (96) can be configured as a disk having a circular boundary (97) and a thickness disposed between two generally planar surfaces (98)(99). The disk shaped printed circuit board (96) can be disposed with the planar surfaces (98)(99) in substantially perpendicular relation to a longitudinal axis (100) of the hollow inside space (89) when configured as a cylindrical volume, as shown in Figures 4 or 5; however, the invention is not so limited, and the components can be mounted on any suitable supporting surface in any configuration or arrangement which allows the components to function as further described below.

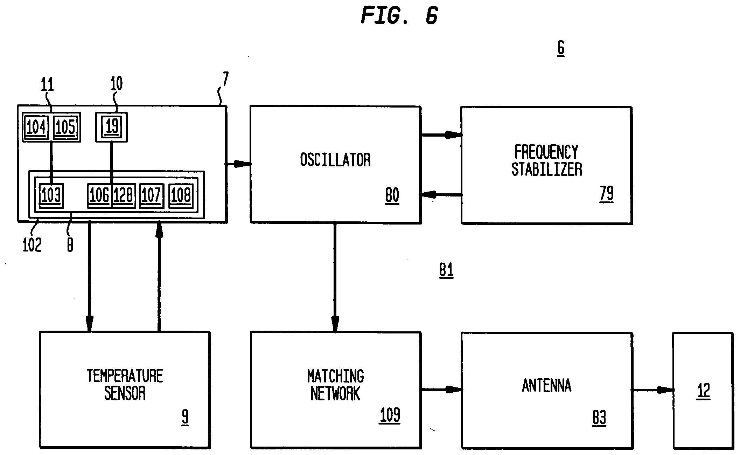

Again referring primarily to Figure 6, which provides a block diagram which represents the various integrated circuit components of the animal monitoring device (6) which function as processing elements, memory elements, logic elements, look-up tables, or the like, to carry out a variety of functions under the control of one or more microprocessors or other control devices, as further described below. In the particular embodiments of the invention shown in Figures 4 through 6, the microcontroller (7) can take the form of a small computer on one or more integrated circuits having one or more processors (8) which control the functions of a variety of processing elements (101) stored in a programmable memory element (102) each of which provides a response to events related to the surveillance, identification, and measurement of values in relation to an individual animal (3) or other object. A microcontroller (7) as available from Microchip Technology. Inc., 2355 West Chandler Blvd., Chandler, Arizona, Part Nos. P1C18LF14K22 or PIC18LF15K22, or similar or equivalent components, can be suitable for use with embodiments of the animal monitoring device (6).

A first processor element (103) can function to encode and continuously or intermittently output an amount of encoded animal identification information (1 I) which can represent a an animal identification value (18) such bolus identification number (104), an animal identification value (105), or other value which associates information received from a bolus (4) to a particular animal (3) or object.

A second processor element (106) can function to intermittently encode and output an amount of encoded sensed animal characteristic information (10) representing a sensed animal characteristic (2) of an animal (3) or object. For the purposes of this invention, an animal characteristic (2) of an animal (3) or object can include any one or more of a physiological characteristics of the animal (3) such as temperature, pH, heart rate, blood pressure, partial pressures of dissolved gases, or the like; or a non-physiological parameter such as animal location, animal tilt, humidity, or the like. The second processor element (106) can in part function to receive analog signals or digital signals from a sensor (9) configured to sense a particular animal characteristic (2). As a non-limiting examples, the sensor (9) (or sensors) can be an omnidirectional tilt and vibration Sensor (PN SQ-SEN-200) distributed by Signal Quest Precision Microsensors; a betachip thermistor (PN 1K2003) distributed by BetaTHERM Sensors; a humidity sensor (PN HCZ-DS) distributed by Ghitron Technology CO., Ltd; an ultra miniature pressure transducer (PN COQ-062) distributed by Kulite, a proximity sensor (PN PY3-AN-3) distributed by Automation Direct.com.

Variation of the sensed animal characteristic(s) (2) can be continuously or intermittently updated by encoding or re-encoding a digital representation of the signal generated by the sensor (9). The second processor element (106) can further function to encode or re-encode from time to time an amount of calibration data (128) which allows calculation and output of a sensed animal characteristic value (19) of the animal (3). As to the particular embodiment of the invention shown in Figures 4 and 5, the second processor element (106) can receive and encode signals received from a thermistor (a type of resistor whose resistance varies with change in temperature). A suitable thermistor for use in embodiment of the invention is available from Microchip Technology, Inc., 2355 West Chandler Blvd., Chandler, Arizona, Part No. MCP98242, and similar and equivalent parts.

A third processor element (107) functions to control the oscillator (80) to generate a stable radio frequency signal (12). An oscillator (80) suitable for use with the invention is available from Freescale Semiconductor, Part No. MC1319x, MC1320x, MC132lx, and MC1322x, and similar or equivalent parts. The third processor element (107) can further function to control a radio frequency stabilizer (82) which functions to offset oscillator (80) wave flux caused by changes in temperature or power to the oscillator (80). A frequency stabilizer (82) suitable for use with the invention is available from Hope Microelectronics Co., Ltd, Part No. HF433E, RF Monolithics, Inc. Part No. RF1172C, and similar or equivalent parts. In regard to the particular embodiment of the invention shown in Figures 4 and 5, the oscillator (80) and frequency stabilizer (82) can generate a radio frequency signal (12) stable between about 410MHz and about 440MHZ. A particular embodiment of the invention generates a radio frequency signal (12) of about 433MHz to be received by the RF reader (13).

A fourth processor element (108) functions to control a network frequency match element (109). The network frequency match element (109) can include capacitors and resistors in combination to deliver a particular radio frequency signal (12) under the conditions of the method utilized (for example the method above described) to the antenna (83). As a non-limiting example, the network frequency match element (109) can detune a 433 MHz radio frequency signal (12) to generate a signal of between about 418-425 MHz. The detuned signal compensates for demodulation of the radio frequency signal (12) due to interaction with the mass of animal (3). The degree of demodulation can be substantially consistent and repeatable from animal (3) to animal (3). Accordingly, the network frequency match element (109) can be configured to compensate for the signal demodulation due to the mass of the animal (3) such that the radio frequency signal (12) transmitted outside of the mass of the animal (3) can be at about 433 MHz (or other selected frequency).

As to particular embodiments, the antenna (83) can be imprinted on the printed circuit board (96) proximate the circular boundary (97) to provide an antenna (83) of generally partial circular configuration having a length of about 37 millimeters and a width of about 1 millimeter (see for example Figures 4 and 5). The antenna (83) operates to transmit the radio frequency signal (12) at the wavelengths above described. An advantage of this configuration of antenna (83) can be that it does not require winding upon or interaction with the magnetic field (110) of the first magnet (85) or one or both of a pair of magnets (95) (or any magnet) to transmit a radio frequency signal (12). Accordingly, this configuration of antenna (83) can provide a lesser amount of interference from the magnetic field (110) of the one or more magnets (85)(95) contained in the bolus (4) resulting a lower incidence of loss of the radio frequency signal (12), less modulation of the radio frequency signal (12) which results in a greater consistency (or lesser amount of lost data) in transmission of animal identification information (11) and sensed animal characteristic information (10).

Again referring to Figures 4 and 5, the bolus (4) can further include a power source (84) located within the hollow inside space (89). The power source (84) shown in Figures 4 and 5 takes the form of a battery (111) such as a AA battery, a AAA battery, or the like. The battery (111) can be inserted or stacked within the hollow inside space (89) proximate the printed circuit board (96). A non-conductive insulator (112) can be disposed between the printed circuit board (96) and the power source (84). The power source (84) provides power to the electronic components supported on the printed circuit board (96). A first battery lead (113) connects the positive battery terminal (109) of the printed circuit board (96) to the positive pole (114) of the battery (111) (or power source) and a second battery lead (115) connects the negative battery terminal (116) of the printed circuit board (96) to the negative pole (116) of the battery (111)(or power source).

Now referring primarily to Figure 4, in particular embodiments of the invention a first non-conductive spacer (117) can be disposed in the hollow inside space (89) of the bolus (4) adjacent to the printed circuit board (96) and a second non-conductive spacer (118) can be disposed in the hollow inside space (89) of the bolus (4) adjacent the battery (111). A first of the pair of magnets (95) can be disposed adjacent the first non-conductive spacer (117) and a second of the pair of magnets (95) can be disposed adjacent the second non-conductive spacer (118). The first of the pair of magnets (94) and the second of the pair of magnets (94) can be configured as magnetic disks or cylinders each having a pair of opposed circular faces disposed a distance apart by the thickness of the magnet. By providing a pair of magnets (94) disposed a distance apart, a first magnetic field (119) generated by the first of the pair of magnets (95) and a second magnetic field (120) generated by the second of the pair of magnets ( ) can attractingly interact with metal objects (121), such as coins, washers, wire, nails, tacks, barbs from barbed wire, or the like, ingested by the animal (3) to magnetically engage these metal objects (121) with the external surface of the bolus (4) such that the metal objects (121) generally align with the longitudinal axis (100) of the bolus (4), for example, substantially the entire length of the metal object (121) can lie against the external surface of the bolus (4) as shown in Figure 4 as opposed to projecting outwardly from the external surface of the bolus (4). Depending upon the configuration of the external surface of the bolus (4), the size, power, and distance separating the first of the pair of magnets (94) and the second of the pair of magnet (95) can be adjusted to correspondingly adjust the interaction of the first magnetic field (119) and the second magnetic field (120) to act on metal objects (121), as above described. For example, in the embodiment of the invention shown in Figure 4, either the particular configuration of the first of the pair of magnets (94) and the second of a pair of magnets (94)(dimensional relations and power) or the particular configuration of the first nonconductive spacer (117) and the second non-conductive spacer (118) can be adjusted to allow metal objects (121) to interact with the external surface of the bolus (4). A second advantage of providing a pair of magnets (94) disposed a distance apart, can be that the printed circuit board (96) can be located between, and a sufficient distance from, either of the pair of magnets (94) to reduce interference with the transmission of the radio frequency signal (12).

Again referring primarily to Figure 4, the printed circuit board (96).supporting the electronic components, the non-conductive insulator (1 12), the non-conductive spacers (117)(118), and the pair of magnets (95) can be overwrapped with a non-conductive wrap element (122) to allow the several elements to moved as a single piece. As one non-limiting example, the non-conductive wrap element (122) can comprise a plastic tube shrinkable in dimension by application of heat to conform the external surface of the components aligned as above described. Accordingly, the overwrapped elements can be inserted into the hollow inside space (89) as a single piece and the at least one end cap (95) can be sealably engaged with first bolus end (90) or second bolus end (91) of the bolus (4). The non-conductive wrap element (122) can have one or more apertures (123). An amount of plastic resin (87), as above described, can flow through the one more apertures to be cast about the components of the animal monitoring device (6).

Now referring primarily to Figure 5, other embodiments of the invention can have a constructional as above described and shown in Figure 4 with the exception of the form and placement of the pair of magnets (94). In the embodiment shown in Figure 5, the pair of magnets (94) and their corresponding magnetic fields (119)(120) along with the nonconductive spacers (117)(118) can be replaced by a first magnet (85) placed adjacent the animal monitoring device (6) and as to those embodiments having a non-conductive wrap element (122) located outside of the non-conductive wrap (122). The animal monitoring device (6) along with the first magnet (85) can be located inside of the inert bolus body (86) whether within an amount of plastic resin (87) or within a scalable container (88) (whether or not the sealable container (88) is also filled with plastic resin (87). As to particular embodiments, the first magnet (85) can have a first and second opposed magnetic faces (123)(124) defining a south pole and a north pole, with the first magnetic face (123) (as to the embodiment shown the south pole) disposed in inward facing relation to the animal monitoring device (6) and the second magnetic face (124)(as to the embodiment shown the north pole) disposed in outward facing relation to said animal monitoring device (6). As to certain preferred embodiments, the first magnet (85) can have a generally rectangular shape having four sides (125) defining the area of a first magnet face (123) (south pole) and the second magnet face (124) (north pole) disposed in substantially parallel opposed relation a distance apart with the first face (123) (south pole) disposed in inward facing relation to the animal monitoring device (6).

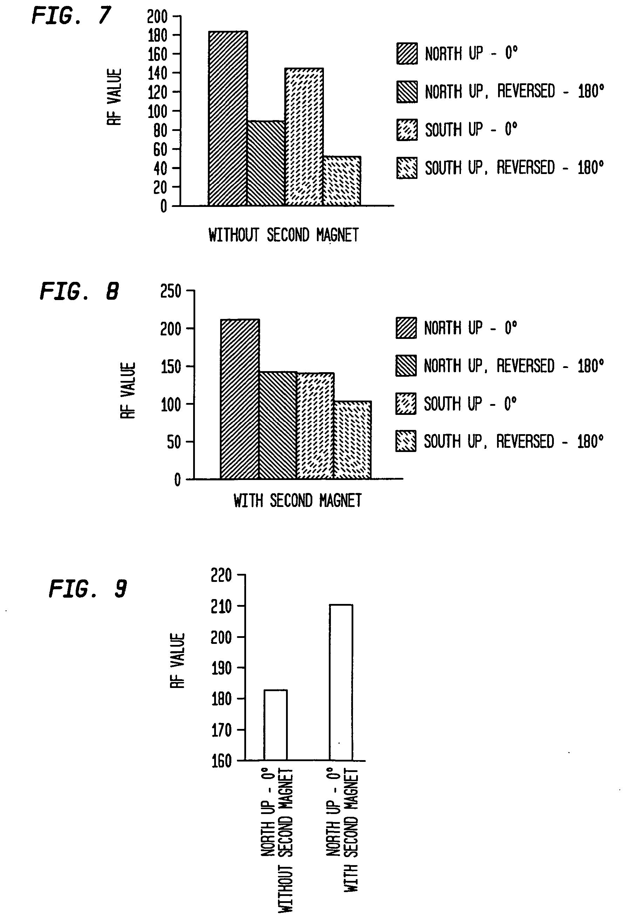

Now referring primary to Figure 7, a bar graph plots the strength of the radio frequency (12) against the orientation of the first magnet (85) in relation to the animal monitoring device (6) located within the inert bolus body (86) (as described for embodiments similar to that shown in Figure 5). Importantly, the orientation of the first magnet (85) in relation to the animal monitoring device (6) can result in a substantial difference in the strength of the received radio frequency signal (12) outside of the bolus (4). Placement of the first magnet (85) with the second magnetic face (124) (north pole) facing outward in relation to the animal monitoring device (6) (north pole designated as "north up" in Figure 7) increases the strength of the received radio frequency signal (12) from the animal monitoring device (6) outside of the bolus (4) as compared to having the first magnetic face (123) (south pole) facing outward in relation to the animal monitoring device (6) (south pole designated as "south up" in Figure 7). Depending upon the type and kind of the first magnet (85), the method in accordance with embodiments of the invention, defines the first magnetic face (123) as the magnetic face which in inward facing relation to the animal monitoring device (6) increases strength of the radio frequency signal (12) received at the radio frequency reader (13). The first magnetic face (123) may define the south pole as described; however, the invention is not so limited, and the first magnetic face (123) may also define the north pole of the first magnet (85), the method selecting the first magnetic face (123) as that face which in the inward facing relation to the animal monitoring device (6) produces the greater strength of radio signal frequency outside of the bolus (4).

Additionally, having placed the first magnetic face (123) (south pole) facing inwardly to increase strength of the received radio signal (12), the first magnet (85) can be rotated to through 180 degrees to find the orientation which further increases the strength of the radio frequency signal (12) outside of the bolus (4). As shown by Figure 7, the first magnetic (85) having the first magnetic face (123) (south pole) facing inwardly in relation to the animal monitoring device (6) and the elongate body of the first magnet (85) substantially aligned with the longitudinal axis (100) of the animal monitoring device (6) is oriented at zero degrees of rotation in relation to the longitudinal axis (100) (as shown in Figure 5). As to this embodiment of the invention, this orientation can produce a substantially increased strength of received radio signal frequency (12) outside of the bolus (4) as compared to having the opposed ends (126)(127) oriented at 180 degrees of rotation in relation to the longitudinal axis (100) (not shown).

Now referring primarily, to Figure 8, embodiments of the invention can further include a second magnet (130) having a location outside of the bolus (4). The second magnet (130) can be orally administered to an animal (3) in similar fashion to the bolus (4). The second magnet (130) can comprise a conventional magnet orally administered to animals (3) to magnetically capture metal objects (121) within the rumen of the animal (3). Particular embodiments of the second magnet (127) can have dimensional relations the same or similar to the first magnet (85) located inside the inert bolus body (86). Interestingly, as shown in Figure 8, magnetic coupling of the second magnet (130) to the first magnet (85) within the bolus (4) can increase the strength of the radio frequency signal (12) outside of the bolus (4), regardless of orientation of the first magnet (85) within the bolus (4), even though the first magnet face (123) (south pole) inwardly facing and in zero degree relation to the longitudinal axis (100) of the animal monitoring device (6) already had the greatest strength of radio frequency signal (12) outside of the bolus (4) (shown as "north up" in Figure 7).

The results set out in the example shown by Figures 7 and 8, was achieved by submerging the bolus (4) of the embodiment shown in Figure 5, and as above described, in an amount of saline solution prepared by dissolving about 27 grams of sodium chloride per liter of water. The bolus (4) submerged in the saline solution was placed about 25 feet from the RF reader (13) to approximate receiving a signal from a bolus (4) within the rumen of a ruminant animal (3) at 75 feet. The bolus (4) between trials was unaltered, except for the orientation of the first magnet (85) in relation to the animal monitoring device (6) contained inside the inert bolus body (86). The first magnet (85) was disposed in a first trial with the north face facing outwardly from the animal monitoring device, and in a second trial with the south face facing outwardly from the animal monitoring device (6). The designation of the first magnetic face (123) of the first magnet (85) was defined by the magnetic face which facing inwardly generates the greatest radio frequency signal (12) received by the RF reader (13). Accordingly, as to the particular embodiment of the invention shown in Figure 5, the south face of the first magnetic (85) faces inwardly toward the animal monitoring device (6) and defines the first magnetic face (123), while the north pole of the first magnet (85) faces outwardly in relation to the animal monitoring device (6) and defines the second magnetic face (124). The first face (123) being defined by the south pole of the first magnet (85), a third trial was conducted in which the first magnet (85) was rotated 180 degrees in relation to the longitudinal axis (100) of the animal monitoring device (6) in reversed relation to the zero degree position. The strength of the radio frequency signal (12) received by the RF reader (13) was determined and the first magnet was place in zero degree or 180 degree relation to the animal monitoring device (6). The results of the trials are set out in the bar graph shown in Figure 7.

The results set out in the example shown by Figure 8, were achieved by submerging the bolus (4) of the embodiment shown in Figure 5 and above described in an amount of saline solution prepared by dissolving about 27 grams of sodium chloride per liter of water. The bolus (4) submerged in the saline solution was placed about 25 feet from the RF reader (13) to approximate receiving a signal from a bolus (4) within the rumen of a ruminant animal (3). As to each trial shown in Figure 7, and described above, an additional trial was conducted by submerging a second magnet (127) in the saline solution in which the bolus (4) containing the first magnet (85) was submerged. In each trial, the second magnet (127) was allowed to magnetically couple the first magnet (85) and the strength of the radio frequency signal (12) was determined. The results being summarized in the bar graph shown in Figure 8. Interestingly, as shown by Figure 9, magnetic coupling of the second magnet (127) with the first magnet (85) increased the strength of the radio frequency signal (12).

The radio frequency signal (12) strength calculated based on the reads gathered by the RF reader (13) during a period of 15 minutes and then multiplied by the signal to noise ratio to produce a RF value utilized to compare strength of radio frequency. As one illustrative example, for a particular bolus if the reads are 2 during the 15 minute period and the signal to noise ratio is 90.7 then the RF value is 181.4.

As can be easily understood from the foregoing, the basic concepts of the present invention may be embodied in a variety of ways. The invention involves numerous and varied embodiments of the method of monitoring an animal within the scope of the appended claims.

As such, the particular embodiments or elements of the invention disclosed by the description or shown in the figures or tables accompanying this application are not intended to be limiting, but rather exemplary of the numerous and varied embodiments generically encompassed by the invention defined by the appended claims. In addition, the specific description of a single embodiment or element of the invention may not explicitly describe all embodiments or elements possible; many alternatives are implicitly disclosed by the description and figures.

Moreover, for the purposes of the present invention, the term "a" or "an" entity refers to one or more of that entity; for example, "a memory element" refers to one or more memory elements. As such, the terms "a" or "an", "one or more" and "at least one" can be used interchangeably herein. Furthermore, a compound "selected from the group consisting of" refers to one or more of the elements in the list that follows, including combinations of two or more of the elements.

All numeric values herein are assumed to be modified by the term "about", whether or not explicitly indicated. For the purposes of the present invention, ranges may be expressed as from "about" one particular value to "about" another particular value. When such a range is expressed, another embodiment includes from the one particular value to the other particular value. The recitation of numerical ranges by endpoints includes all the numeric values subsumed within that range. A numerical range of one to five includes for example the numeric values 1, 1.5, 2, 2.75, 3, 3.80, 4, 5, and so forth. It will be further understood that the endpoints of each of the ranges are significant both in relation to the other endpoint, and independently of the other endpoint. When a value is expressed as an approximation by use of the antecedent "about," it will be understood that the particular value forms another embodiment. The term "about" generally refers to a range of numeric values that one of skill in the art would consider equivalent to the recited numeric value or having the same function or result.

Moreover, for the purposes of the present invention, the term "a" or "an" entity refers to one or more of that entity unless otherwise limited. As such, the terms "a" or "an", "one or more" and "at least one" can be used interchangeably herein.

## Claims

1. A method of monitoring an animal (3), comprising the steps of:
a) orally administering to a ruminant animal (3) a monitoring device (6) and a first magnet (85) contained inside of an inert bolus body (86), said animal monitoring device (6) including:
i) at least one sensor (9) capable of generating a signal which varies in relation to change in a sensed animal characteristic (2);
ii) a signal encoder (106) which encodes said signal generated by said sensor as encoded sensed animal characteristic information (10);
iii) a radio frequency signal generator (81) which generates a radio frequency signal (12) capable of carrying said encoded sensed animal characteristic information(10);
iv) a power source (84) which supplies power to said animal monitoring device (6); and
v) an antenna (83) capable of transmitting said radio frequency signal (12) carrying said encoded sensed animal characteristic information (10); and
b) accessing said encoded sensed animal characteristic information (10) carried by said radio frequency signal (12);
wherein said radio frequency generator (81) comprises an oscillator (80) which generates said radio frequency signal (12) and a network frequency match element (109) which compensates for demodulation of said radio frequency signal (12) passing through the mass of said ruminant animal (3).

2. The method of monitoring an animal of claim 1, further comprising the step of orally administrating to said ruminant animal (3) a second magnet separate from said first magnet (85) contained inside of said inert bolus body (86), said second magnet adapted to magnetically couple said first magnet (85) contained inside of said inert bolus body (86) to increase transmission of said radio frequency signal (12) capable of carrying said encoded animal identification information (11) and said encoded sensed animal characteristic information (10).

3. The method of monitoring an animal of claim 1, wherein said encoded sensed animal characteristic information (10) is selected from the group consisting of: temperature, pH, heart rate, blood pressure, and partial pressures of dissolved gases, or combinations thereof.

4. The method of monitoring an animal of claim 3, wherein said monitoring device (6) further includes an animal identification information encoder (103) which encodes animal identification information related to said sensed animal characteristic as encoded animal identification information (11), and further comprising the steps of:
a) accessing said encoded animal identification information (11) carried by said radio frequency signal (12); and
b) matching said encoded animal identification information (11) with said encoded sensed animal characteristic information (10) carried by said radio frequency signal (12).

5. The method of monitoring an animal of claim 4, further comprising the step of locating one or more radio frequency readers (13) capable of receiving said radiofrequency signal (12) carrying said encoded animal identification information (11) and said encoded sensed animal characteristic information (10) at a distance from said animal monitoring device (6).

6. The method of monitoring an animal of claim 5, further comprising the step of transmitting said encoded animal identification information (11) and said encoded sensed animal characteristic information (10) from said one or more radio frequency readers (13).

7. The method of monitoring an animal of claim 6, further comprising the step of receiving said encoded animal identification information (11) and said encoded sensed animal characteristic information (10) from said one or more radio frequency readers (13) with a reception device (64).

8. The method of monitoring an animal of claim 7, further comprising the step of using a computer (17) configured to receive and provide a user (20) access to said encoded animal identification information (11) and said encoded sensed animal characteristic information (10) received by said reception device (64).

9. The method of monitoring an animal of claim 1, wherein said radio frequency generator (81) further comprises a radio frequency stabilizer (82) which operates to maintain said radio frequency signal (12) within a radio frequency range.

10. The method of monitoring an animal of claim 9, wherein said radio frequency stabilizer (82) maintains said radio frequency signal (12) in the range of 410MHz and 440MHz.

11. The method of monitoring an animal of claim 1, wherein said animal monitoring device (6) further includes a microcontroller (7) which controls one or more of said sensor signal encoder (106), said animal identification information encoder (103), and said radio frequency signal generator (81).

12. The method of monitoring an animal of claim 11, wherein said animal monitoring device (6) further includes a printed circuit board (96) which supports and electrically connects one or more of said microcontroller (7), said sensor signal encoder (103), said animal identification information encoder (106) said radio frequency signal generator (81), and said antenna (83) with each other.

13. The method of monitoring an animal of claim 12, wherein said printed circuit board (96) defines a circular boundary (97) having said antenna (83) imprinted proximate said circular boundary (97).

## Patentansprüche

1. Verfahren zum Überwachen eines Tieres (3), umfassend die Schritte:
a) orales Verabreichen einer Überwachungsvorrichtung (6) und eines ersten Magneten (85), der im Inneren eines inerten Boluskörpers (86) enthalten ist, an ein wiederkäuendes Tier (3), wobei die Tierüberwachungsvorrichtung (6) beinhaltet:
i) zumindest einen Sensor (9), der in der Lage ist, ein Signal zu erzeugen, das sich in Abhängigkeit von der Änderung eines erfassten Tiermerkmals (2) ändert;
ii) einen Signalkodierer (106), der das von dem Sensor erzeugte Signal als kodierte erfasste Tiermerkmalsinformation (10) kodiert;
iii) einen Hochfrequenzsignalgenerator (81), der ein Hochfrequenzsignal (12) erzeugt, das in der Lage ist, die kodierte erfasste Tiermerkmalsinformation (10) zu übertragen;
iv) eine Energiequelle (84), die die Tierüberwachungsvorrichtung (6) mit Energie versorgt; und
v) eine Antenne (83), die in der Lage ist, das Hochfrequenzsignal (12) zu übertragen, das die kodierte erfasste Tiermerkmalsinformation (10) trägt; und
b) Zugreifen auf die kodierte erfasste Tiermerkmalsinformation (10), die von dem Hochfrequenzsignal (12) getragen wird;
wobei der Hochfrequenzgenerator (81) einen Oszillator (80), der das Hochfrequenzsignal (12) erzeugt, und ein Netzwerk-Frequenzanpassungselement (109) umfasst, das die Demodulation des Hochfrequenzsignals (12) kompensiert, das durch die Masse des wiederkäuenden Tiers (3) hindurchgeht.

2. Verfahren zum Überwachen eines Tieres nach Anspruch 1, das ferner den Schritt des oralen Verabreichens eines zweiten Magneten, der von dem ersten Magneten (85), welcher in dem inerten Boluskörper (86) enthalten ist, getrennt ist, an das wiederkäuende Tier (3) umfasst, wobei der zweite Magnet geeignet ist, den ersten Magneten (85), der in dem inerten Boluskörper (86) enthalten ist, magnetisch zu koppeln, um die Übertragung des Hochfrequenzsignals (12) zu erhöhen, das in der Lage ist, die kodierte Tieridentifikationsinformation (11) und die kodierte erfasste Tiermerkmalsinformation (10) zu tragen.

3. Verfahren zum Überwachen eines Tieres nach Anspruch 1, wobei die kodierte erfasste Tiermerkmalsinformation (10) ausgewählt ist aus der Gruppe bestehend aus: Temperatur, pH-Wert, Herzfrequenz, Blutdruck und Partialdrücken gelöster Gase, oder Kombinationen davon.

4. Verfahren zum Überwachen eines Tieres nach Anspruch 3, wobei die Überwachungsvorrichtung (6) ferner einen Tieridentifikationsinformationskodierer (103) beinhaltet, der Tieridentifikationsinformation, die sich auf das erfasste Tiermerkmal bezieht, als kodierte Tieridentifikationsinformation (11) kodiert, und das des Weiteren die folgenden Schritte umfasst:
a) Zugreifen auf die kodierte Tieridentifikationsinformation (11), die von dem Hochfrequenzsignal (12) getragen wird; und
b) Abgleichen der kodierten Tieridentifikationsinformation (11) mit der kodierten erfassten Tiermerkmalsinformation (10), die von dem Hochfrequenzsignal (12) getragen wird.

5. Verfahren zum Überwachen eines Tieres nach Anspruch 4, des Weiteren umfassend den Schritt des Anordnens eines oder mehrerer Hochfrequenzlesegeräte (13), die in der Lage sind, das Hochfrequenzsignal (12) zu empfangen, welches die kodierte Tieridentifikationsinformation (11) und die kodierte erfasste Tiermerkmalsinformation (10) trägt, in einer Entfernung von der Tierüberwachungsvorrichtung (6).

6. Verfahren zum Überwachen eines Tieres nach Anspruch 5, des Weiteren umfassend den Schritt des Übertragens der kodierten Tieridentifikationsinformation (11) und der kodierten erfassten Tiermerkmalsinformation (10) von dem einen oder den mehreren Hochfrequenzlesegeräten (13).

7. Verfahren zum Überwachen eines Tieres nach Anspruch 6, des Weiteren umfassend den Schritt des Empfangens der kodierten Tieridentifikationsinformation (11) und der kodierten erfassten Tiermerkmalsinformation (10) von dem einen oder den mehreren Hochfrequenzlesegeräten (13) mit einer Empfangsvorrichtung (64).

8. Verfahren zum Überwachen eines Tieres nach Anspruch 7, des Weiteren umfassend den Schritt des Verwendens eines Computers (17), der eingerichtet ist, die kodierte Tieridentifikationsinformation (11) und die kodierte erfasste Tiermerkmalsinformation (10), die von der Empfangsvorrichtung (64) empfangen werden, zu empfangen und einem Benutzer (20) Zugriff darauf zu gewähren.

9. Verfahren zum Überwachen eines Tieres nach Anspruch 1, wobei der Hochfrequenzgenerator (81) des Weiteren einen Hochfrequenzstabilisator (82) umfasst, der so arbeitet, dass das Hochfrequenzsignal (12) innerhalb eines Hochfrequenzbereichs gehalten wird.

10. Verfahren zum Überwachen eines Tieres nach Anspruch 9, wobei der Hochfrequenzstabilisator (82) das Hochfrequenzsignal (12) im Bereich von 410 MHz und 440 MHz hält.

11. Verfahren zum Überwachen eines Tieres nach Anspruch 1, wobei die Tierüberwachungsvorrichtung (6) des Weiteren einen Mikrocontroller (7) beinhaltet, der einen oder mehrere von dem Sensorsignalkodierer (106), dem Tieridentifikationsinformationskodierer (103) und dem Hochfrequenzsignalgenerator (81) steuert.

12. Verfahren zum Überwachen eines Tieres nach Anspruch 11, wobei die Tierüberwachungsvorrichtung (6) des Weiteren eine Leiterplatte (96) beinhaltet, die den Mikrocontroller (7), den Sensorsignalkodierer (103), den Tieridentifikationsinformationskodierer (106), den Hochfrequenzsignalgenerator (81) und die Antenne (83) trägt und elektrisch miteinander verbindet.

13. Verfahren zum Überwachen eines Tieres nach Anspruch 12, wobei die gedruckte Leiterplatte (96) eine kreisförmige Begrenzung (97) definiert und die Antenne (83) aufweist, aufgedruckt in der Nähe der kreisförmigen Begrenzung (97).

## Revendications

1. Procédé pour surveiller un animal (3), comprenant les étapes consistant à :
a) administrer oralement à un animal ruminant (3) un dispositif de surveillance (6) et un premier aimant (85) contenu à l'intérieur d'un corps de bolus inerte (86), ledit dispositif de surveillance d'animal (6) comprenant :
i) au moins un capteur (9) capable de générer un signal qui varie en fonction du changement d'une caractéristique détectée (2) de l'animal ;
ii) un codeur de signaux (106) qui code ledit signal généré par ledit capteur en tant qu'informations caractéristiques d'animal détectées codées (10) ;
iii) un générateur (81) de signaux radioélectriques qui génère un signal radioélectrique (12) capable de porter lesdites informations caractéristiques d'animal détectées codées (10) ;
iv) une source d'énergie (84) qui fournit de l'énergie audit dispositif de surveillance d'animal (6) ; et
v) une antenne (83) capable d'émettre ledit signal radioélectrique (12) portant lesdites informations caractéristiques d'animal détectées codées (10) ; et
b) accéder auxdites informations caractéristiques d'animal détectées codées (10) portées par ledit signal radioélectrique (12) ;
dans lequel ledit générateur de radiofréquence (81) comprend un oscillateur (80) qui génère ledit signal radioélectrique (12) et un élément d'adaptation de fréquence de réseau (109) qui compense la démodulation dudit signal radioélectrique (12) passant à travers la masse dudit animal ruminant (3).

2. Procédé pour surveiller un animal selon la revendication 1, comprenant en outre l'étape consistant à administrer oralement audit animal ruminant (3) un deuxième aimant séparé dudit premier aimant (85) contenu à l'intérieur dudit corps de bolus inerte (86), ledit deuxième aimant étant adapté pour coupler magnétiquement ledit premier aimant (85) contenu à l'intérieur dudit corps de bolus inerte (86) pour augmenter la transmission dudit signal radioélectrique (12) capable de porter lesdites informations d'identification d'animal codées (11) et lesdites informations caractéristiques d'animal détectées codées (10).

3. Procédé pour surveiller un animal selon la revendication 1, dans lequel lesdites informations caractéristiques d'animal détectées codées (10) sont choisies dans le groupe constitué par : la température, le pH, la fréquence cardiaque, la tension artérielle et les pressions partielles de gaz dissous ou des associations de telles informations.

4. Procédé pour surveiller un animal selon la revendication 3, dans lequel ledit dispositif de surveillance (6) comprend en outre un codeur d'informations d'identification d'animal (103) qui code des informations d'identification d'animal liées à ladite caractéristique d'animal détectée en tant qu'informations d'identification d'animal codées (11), et comprenant en outre les étapes consistant à :
a) accéder auxdites informations d'identification d'animal codées (11) portées par ledit signal radioélectrique (12) ; et
b) mettre en correspondance les informations d'identification d'animal codées (11) avec lesdites informations caractéristiques d'animal détectées codées (10) portées par ledit signal radioélectrique (12).

5. Procédé pour surveiller un animal selon la revendication 4, comprenant en outre l'étape consistant à localiser un ou plusieurs lecteurs de radiofréquence (13) capables de recevoir ledit signal radioélectrique (12) portant lesdites informations d'identification d'animal codées (11) et lesdites informations caractéristiques d'animal détectées codées (10) à distance dudit dispositif de surveillance d'animal (6).

6. Procédé pour surveiller un animal selon la revendication 5, comprenant en outre l'étape consistant à transmettre lesdites informations d'identification d'animal codées (11) et lesdites informations caractéristiques d'animal détectées codées (10) à partir dudit un ou desdits plusieurs lecteurs de radiofréquence (13).

7. Procédé pour surveiller un animal selon la revendication 6, comprenant en outre l'étape consistant à recevoir lesdites informations d'identification d'animal codées (11) et lesdites informations caractéristiques d'animal détectées codées (10) à partir dudit un ou desdits plusieurs lecteurs de radiofréquence (13) avec un dispositif de réception (64).

8. Procédé pour surveiller un animal selon la revendication 7, comprenant en outre l'étape consistant à utiliser un ordinateur (17) configuré pour recevoir et fournir à un utilisateur (20) l'accès auxdites informations d'identification d'animal codées (11) et auxdites informations caractéristiques d'animal détectées codées (10) reçues par ledit dispositif de réception (64).

9. Procédé pour surveiller un animal selon la revendication 1, dans lequel ledit générateur de radiofréquence (81) comprend en outre un stabilisateur de radiofréquence (82) qui fonctionne pour maintenir ledit signal radioélectrique (12) dans une gamme de radiofréquences.

10. Procédé pour surveiller un animal selon la revendication 9, dans lequel ledit stabilisateur de radiofréquence (82) maintient ledit signal radioélectrique (12) dans la gamme de 410 MHz à 440 MHz.

11. Procédé pour surveiller un animal selon la revendication 1, dans lequel ledit dispositif de surveillance d'animal (6) comprend en outre un microcontrôleur (7) qui commande un ou plusieurs dudit codeur de signaux de capteur (106), dudit codeur d'informations d'identification d'animal (103) et dudit générateur de signaux radioélectriques (81).

12. Procédé pour surveiller un animal selon la revendication 11, dans lequel ledit dispositif de surveillance d'animal (6) comprend en outre une carte de circuit imprimé (96) qui supporte et connecte électriquement un ou plusieurs dudit microcontrôleur (7), dudit codeur de signaux de capteur (103), dudit codeur d'informations d'identification d'animal (106), dudit générateur de signaux radioélectriques (81) et de ladite antenne (83) les uns avec les autres.

13. Procédé pour surveiller un animal selon la revendication 12, dans lequel ladite carte de circuit imprimé (96) définit une limite circulaire (97) ayant ladite antenne (83) imprimée à proximité de ladite limite circulaire (97).
